# EUROPEAN PATENT APPLICATION

(11) **EP 2 821 144 A1**
(43) Date of publication of application: **07.01.2015**
(21) Application number: 13174602.6
(22) Date of filing: 02.07.2013
(51) Int. Cl.: B04B 1/00, B04B 5/04, B04B 5/10, B04B 7/12, B04B 11/06, C12M 1/00, C12M 1/04, C12M 1/12, C12M 3/04

(54) **Centrifugation chamber with gas-permeable membrane for cell cultivation**

(71) Applicant: Miltenyi Biotec GmbH, 51429 Bergisch Gladbach (DE)
(72) Inventor: Kabaha Eiad Dr., 53229 Bonn (DE); Peters Ralf-Peter Dr., 51467 Bergisch Gladbach (DE); Miltenyi Stefan, 51789 Bergisch Gladbach (DE)
(74) Representative: Kisters, Michael Marcus

(57) **Abstract**

The invention is directed to a centrifuge chamber comprising a cylinder (5)having a base plate (12) and a cover plate (11), a rotational axis with at least one port (10) for input and/or output of liquids, at least one port (3) for input of gases and at least one layer for cell culturing, wherein the layer for cell culturing comprises a gas-permeable membrane (1) on which cells can be cultured and wherein the at least one port (3) for input and/or output of gases is connected to the gas-permeable membrane (1).

## Description

The invention relates to a chamber for a centrifuge provided with a gas-permeable membrane on which cells can be cultivated and a process for cell culture using this chamber.

### Background of the Invention

Fractionation and separation of cells from suspensions like blood or bone marrow is becoming more and more part of medical treatment. For such treatment, cells are extracted from a patient, then separated to provide the desired target cells, which are usually stimulated/manipulated/expanded by cell culturing before introducing into the same or a different patient. Extracting, preparation, fractionation, separation, manipulation and introducing of the cells should be performed as fast as possible to reduce stress imposed on the target cells and the patients. Contrary to these requirements, cell culturing is a laborious and time-consuming process.

Fractionation and separation of cell suspensions by centrifugation is long known in the art to separate samples of biological origin into two or more components. For example, the separation of human blood into leucocytes, erythrocytes and blood plasma is an often performed process and can be conduced in a semi automated apparatuses as disclosed in US 6605223, US 4446014 or US 81242342. These publications are silent about cell culturing.

WO 2009/072006 and WO 2009/072003 disclose a centrifugation system with centrifugation chambers suitable for cell separation. The chamber can be provided with layers for adhering and culturing the separated cells. WO 2009/072006 and WO 2009/072003 are silent about the cell culturing conditions and the supply of the cells with gases and nutrition. This centrifugation system and/or centrifugation chambers is optimized for cell separation rather than cell culturing of the separated cells.

Since new cell therapies require not only separation of target cells, but also expansion of the target cells to provide a sufficient number of cells, it is an object of the invention to provide a centrifugation chamber which allows cell separation and cell culture in the same centrifugation chamber and in an automated process. A crucial point in cell culture is the supply of the cells with gas like oxygen or carbon dioxide.

Object of the present invention is to provide a centrifuge chamber enabling cell cultivation and cell separation in a single container without manual manipulation of the cells.

### Object of the invention

Accordingly it is an object of the invention to provide a centrifuge chamber comprising a cylinder having a base plate and a cover plate, a rotational axis with at least one port for input and/or output of liquids, at least one port for input of gases and at least one layer for cell culturing, wherein the layer for cell culturing comprises a gas-permeable membrane on which cells can be cultured and wherein the at least one port for input of gases is connected to the gas-permeable membrane.

Cell culturing on the gas-permeable membrane according to the invention relates to all methods where cells are kept physiologically active and optionally are modified. The modification may result for example in a change of the phenotype, function, number or differentiation status of the cells. Cell culturing on the gas-permeable membrane according to the invention may lead to
a) cell division, differentiation or cell proliferation
b) activation of a signal transduction cascade
c) change of the cellular activation status and/or cell function
d) genetic modification of cells
e) growing of layers of different or identical cell types involving cell-cell contact

In the centrifuge chamber according to the invention, the gas-permeable membrane can be applied on a support structure or on the base plate. Accordingly, the gas-permeable membrane has a first surface on which the cells are cultured ("cell-side") and a second surface, to which the gases are supplied ("gas-side"). To supply the cells, gas migrates from the gas-side of the membrane to the cell-side.

The gas-permeable membrane may be applied on the base plate of the chamber or on a support structure. At least one port for input of gases of the chamber is connected to the gas-permeable membrane, which means that gases can be supplied to the volume between the gas-permeable membrane and the base plate or to the volume between the gas-permeable membrane and the support structure.

### Prior Art

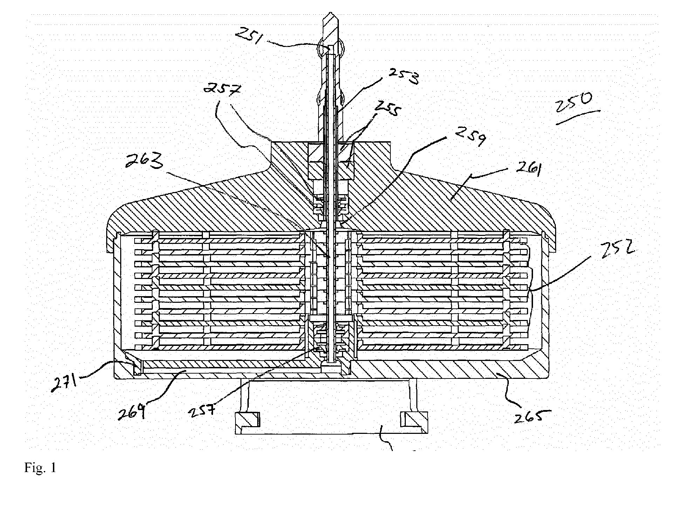

Fig. 1 shows a standard centrifuge chamber according to the prior art, for example as disclosed in WO 2009/072006 or WO 2009/072003. This centrifuge chamber is provided with two input/output ports in the axis of the chamber connected via tubes to openings into the chamber and layers for cell culture. The cells are supplied with gas and cell culture media via the input/output ports and by diffusion through the liquid in the whole chamber. The supply of the cells with gases in such system will be insufficient and/or inhomogeneous since cells at the periphery of the layers or adjacent to the input/output ports will are better supplied than cells adhered more towards the rotational axis.

### General description of the centrifugation chamber of the invention

The chamber according to the invention comprises a circular base plate and a circular cover plate, both of which are oriented substantially perpendicular to a rotational axis; and a cylinder or a wall which is oriented substantially perpendicular to the base and cover plate such that base plate, cover plate and cylinder can be glued or welded together in a water and gas-tight fashion. Thereby, a closed centrifugation chamber is formed, consisting of a pot-like bottom part and an upper part in the form of a lid.

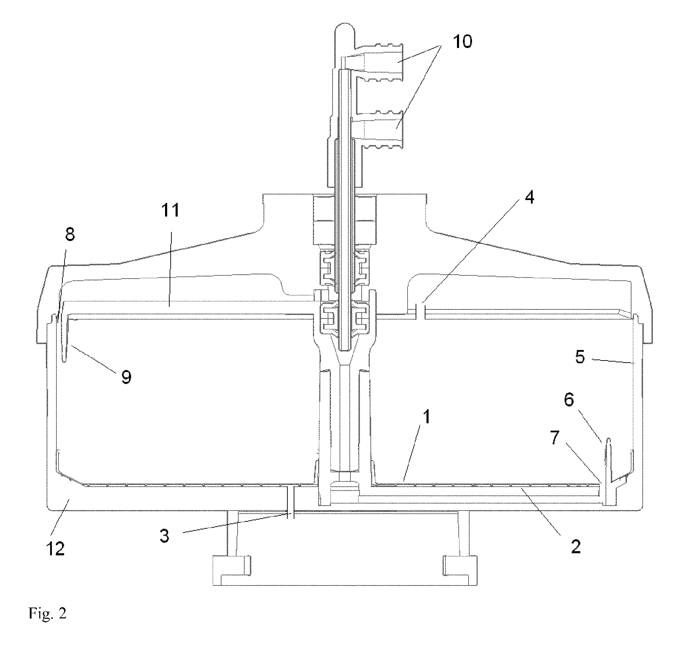

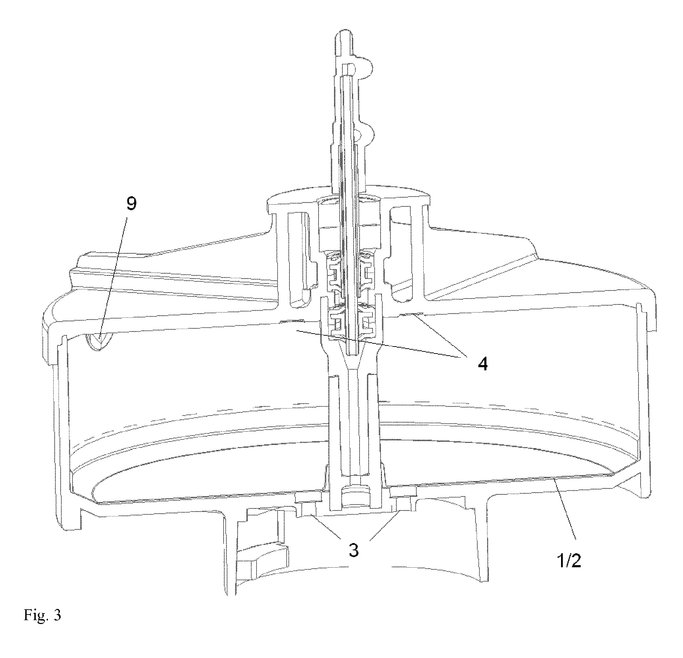

Fig. 2 and 3 show by way of example centrifugation chambers according to the invention. These chambers comprise a gas-permeable membrane (1) as layer for cell culturing applied on a support structure (2) or the base plate (12). Gas necessary to supply the cells are distributed by diffusion through gas-permeable membrane (1) and are homogeneously distributed over the whole area of the membrane. The gases can enter the chamber via at least one port (3) to the volume between the membrane (1) and the support (2). Gas which is not consumed by the cells may exit the chamber via port (4) in the cover plate.

The centrifugation chamber according to the invention can be used as or in a cell processing system and/or the process of the invention. For example, cell processing according to the invention can be performed by first expanding a sample of cells by cell culturing. The resulting cell suspension may the subsequently be separated into two or more fractions by centrifugation. The separation process may involve one or more washing steps and the desired target cells may be expanded in an additional culturing step.

In another variant, cell processing according to the invention involves enrichment of the desired target for a cell sample cells in a first centrifugation step including one or more washing steps to remove unwanted cells. The thus enriched cell fraction comprises the desired cells which can then be expanded by a subsequent cell culturing step.

The centrifugation chamber according is especially suitable for processing cells originating from blood or bone marrow, like leukapheresis samples or alike.

### Detailed description of the centrifugation chamber of the invention

### Gas permeable membrane

The gas-permeable membrane according to the invention serves as layer for cell culturing. Supply of the cells in the centrifuge chamber of the invention is achieved by gas diffusion through the membrane.

The gas-permeable membrane may be manufactured from polystyrene, silicone like SILPURAN 6000/50 or SILPURAN 2450 (Wacker Chemie AG), polymethylpentene or carbon film and may by glued, welded or press-formed on the support structure or on the base plate of the chamber in a water-tight and gas-tight manner.

In order to enhance the homogeneous distribution of the gases to the gas-permeable membrane, the gas-permeable membrane can be provided with a plurality of spacer elements or channels on the gas-side. The spacer elements or channels of the permeable membrane ensure sufficient volume and/or a defined space between the membrane and the support structure or the membrane and the base plate to enhance the distribution of gases over the surface of the membrane.

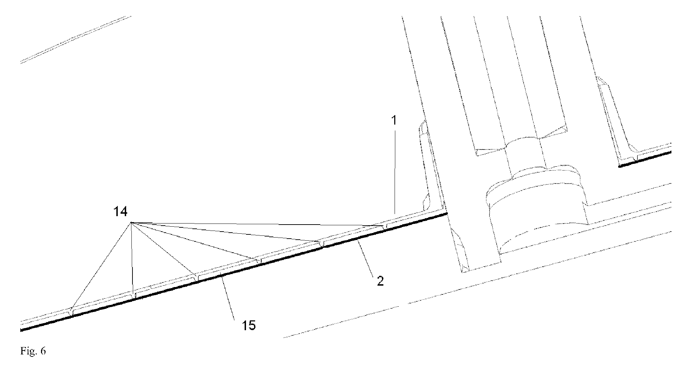

Spacer elements may have the form of dots, textures or tappets or the like and have a height of 0.1 to 0.5 mm. The channels can have a depth and width of 0.1 to 0.5 mm and can be arranged in any geometrical form like spiral shaped, maze or labyrinth-like fashion. Fig. 6 shows by way of example a gas-permeable membrane with a plurality of spacer elements (14) to provide a volume (15) between the membrane and the support structure (2). Of course, the channels of spacer elements are oriented between the gas-permeable membrane and the support structure or the base plate and are not in contact with the cells.

The gas-permeable membrane has preferable an optical transparence suitable for optical microscopy, i.e. has a haze and light transmittance comparable to glass. The surface of the gas-permeable membrane should be a as smooth as possible for this purpose.

### Support structure

The optional support structure gives the gas-permeable membrane sufficient mechanical stability under centrifugation conditions and provides a defined volume under the membrane. The support structure is located on or over the base plate. In alternative to a support structure, the base plate may be used al support for the gas-permeable membrane.

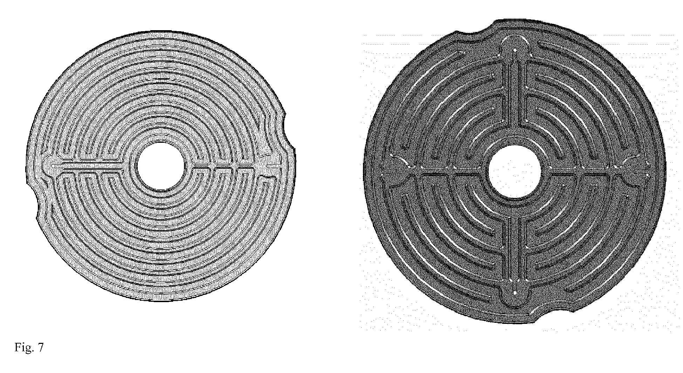

In order to enhance the homogeneous distribution of the gases to the gas-permeable membrane, the support structure can be provided with a plurality of spacer elements or channels. Spacer elements may have the form of dots, textures or tappets or the like and have a height o f 0.1 to 0.5 mm. The channels can have a depth and width of 0.1 to 0.5 mm and can be arranged in any geometrical form like spiral shaped, maze or labyrinth-like fashion. Fig. 7 shows by way of example two support structures with channels.

The support structure has preferable an optical transparence suitable for optical microscopy, i.e. has a haze and light transmittance comparable to glass. The surfaces of the support structure should be a as smooth as possible for this purpose.

### Layer for cell culturing

Gas-permeable membrane and the support structure form a layer for cell culturing. This layer can be fixed at the rotational axis of the chamber. Hereby, the layer is subjected to the same rotational speed as the chamber/the rotational axis. In a first variant of this embodiment of the invention, the cells adhere to the membrane and can be cultivated on the layer at low rotational speed of the chamber. At higher speeds of rotation, the cells are removed by the centrifugal forces from the membrane and are suspended in the cell culturing media. The suspended cell can be separated from each other by centrifugation of the chamber according to their respective speed of sedimentation.

In a second variant of this embodiment of the invention, the layer is not fixed at the rotational axis of the chamber, but can rotate freely about the rotational axis of the chamber. In this variant of the invention, the cell media is distributed through the chamber by rotation of the layer relative to the chamber. The layer will not remain idle, but will rotate in a slow manner similar to the movement of a roller fermenter.

In a third variant of this embodiment of the invention, the layer is not tightly fixed at the rotational axis of the chamber, but can rotate for about a quarter to half of a turn until further free rotation is blocked. In this variant, the chamber may change its direction of rotation frequently and the cells are supplied by cell media is distributed by the movement of the chamber. At higher speeds of rotation, the free rotation of the layer is blocked and the cells are removed from the surface due to shear forces. After being dispersed in the cell culturing media, the cells can be separated under centrifugal forces according to their respective speed of sedimentation.

### Centrifugation chamber

The chamber may have an inner diameter of 2 cm to 20 cm, preferable 8 to 15 cm and a inner height of 5 mm to 10 cm, preferable 2 cm to 7 cm. The total volume of the chamber can be between 10 cm³ and 2000 cm³, preferable between 200 cm³ and 1000 cm³.

The chamber of the invention consists of a cylinder, a base and a cover plate. Cylinder, base and cover plate can be round or circular objects in order to simplify production and to reduce any imbalance during the centrifugation process. In another embodiment, cylinder, base and cover plate may be shaped slightly elliptic, with a diameter in a first dimension being 0.5 to 10, preferable 0.5 to 5% larger than in a second dimension orientated perpendicular to the first dimension. For example the cylinder may have in a first dimension a diameter of 120 mm and in a second dimension being orientated perpendicular to the first dimension a diameter of 122 mm. The base and cover may have the same elliptic shape as the cylinder or can be round / circular objects.

The chamber and the support structures may be made of various materials like ceramics, polystyrene (PS), polyethylene (PE), polypropylene (PP), polyvinylchloride, polycarbonate, glass, polyacrylate, polyacrylamide, polymethylmethacrylate (PMMA), and/or polyethylenterephtala (PET), Polytetrafluorethylen (PTFE) and /or thermoplastic polyurethane (TPU), silicone or compositions comprising one or more of the above mentioned materials. Furthermore, the chamber may comprise or by made of biodegradable material like collagen, chitin, alginate, and/or hyaluronic acid derivatives, polyactide (PLA), polyvinyl alcohol (PVA), polyglycolida (PGA) and their copolymers.

Cell processing according to the invention involves separation of the cell suspension into two or more fractions by centrifugation. After separating the cell fractions, unwanted liquids or cell suspensions are removed from the chamber via openings in the base plate and/or cover plate and through the input/output ports. A cell separation step may optionally comprise one or more washing steps wherein washing liquids are separated from the cell suspension centrifugation and are subsequently removed via the openings and the input/output ports of liquids of the chamber.

Addition and removal of cell suspension, nutrition liquids and/or washing liquids from/to the centrifugation chamber is possible via the ports for input/output of liquids which are connected to appropriate openings in the chamber.

The openings of the chamber (for example (7) and (8) in Fig. 2) can be shaped as holes or line entries and their position in the centrifugation chamber can be configured such that they are best suited for the separation of a particular sample or for the draining of particular fluids in or out of the chamber. Depending on the components of a particular sample and the relative volume of each component in the sample, the openings can be positioned in a way that the fastest removal and/or detection of a particular layer can be achieved. In addition, the size of the openings can be optimized for the desired layer, for example in view of the size of the target cells and/or optimized volume flow.

In case the cylinder is shaped slightly elliptic, it is preferred that the openings with deflectors are oriented in the larger dimension of the elliptic cylinder. In this case, the cells will be moved by the force of centrifugation along the cylinder walls in the direction of the larger dimension of the cylinder i.e. in vicinity to the openings.

In a further embodiment, the chamber according to the invention comprises a means for controlling the progress of the sample separation, positioned at the base plate or the cover plate of the chamber. The means for controlling the progress of the sample separation is preferably positioned at a channel or at a gap located in the base plate or the cover plate of the chamber such that the sample can enter the channel or gap during the centrifugation and thus becomes detectable. Due to the centrifugal force, different components of the sample will form layers, which are detectable by light, for example with a camera or a light detector. Thereby, a signal is generated that allows for determining when the layer formation or sample separation is complete. Suitable means for controlling the progress of the sample separation are disclosed in WO 2009/072006 or WO 2009/072003 incorporated herein by reference.

The centrifugation chamber can be used for cell separation processes, for cell culture purposes and for further processing of the cells grown therein. The chamber allows a large range of cell culture methods to be performed, such as growing of cells, separating, washing, enriching the cells or different kinds of cells, or others. For this purpose, the chamber may comprise further inlet/outlet openings, e.g. for gas, cell culture media or alike. Cell culture conditions are known in the art.

The cell culturing process with the chamber of the invention with or without centrifugation conditions is performed until the desired numbers of target cells is achieved or the capacity of the layer is exhausted. The duration of the process depends on the desired target cells and is not limited. Typically, the process for cell culturing may take between 1 and 24 hours.

Centrifugation is preferentially carried out between 10 and 2000 x g, preferable between 100 and 500 x g. In a preferred embodiment, the chamber can be heated or cooled to provide a temperature appropriate for cell culturing. For this purpose, a heating and/or cooling means can be located at the chamber or surrounding the chamber.

### Chamber with deflectors for enhanced separation process

The overall cell processing process can be enhanced or accelerated by modifications of the chamber, especially by providing the openings of the input/output ports of liquids into the chamber with deflectors.

In another embodiment of the centrifuge chamber according to the invention, at least one port for input and/or output of liquids is connected to openings located in the base plate and/or cover plate into the centrifuge chamber and at least one opening is provide with at least one deflector having a width at its base of at most 1/10 of the inner circumference of the cylinder. In a first embodiment of the invention, at least one deflector is located between at least one opening and the cylinder. Located here, this deflector shields the opening from the volume between opening and cylinder and prevents during draining of this volume the unwanted sucking of liquid from another part of the chamber i.e. from the volume between the opening and the cylinder of the chamber. This embodiment is shown by way of example in Fig. 2 with opening (7) and deflector (6). In this embodiment, the chamber may be provided with 2, 4, 6 or 8 openings in both the base and cover plate.

In a second embodiment, at least one deflector is located between at least one opening and the rotational axis of the cylinder. Different from the first embodiment, this deflector shields the opening from the volume of the chamber between opening and cylinder wall and prevents sucking liquid from this volume when draining the internal volume of the chamber. This embodiment is shown by way of example in Fig. 2 with opening (8) and deflector (9). In this embodiment, the chamber may be provided with 2, 4, 6 or 8 openings in both the base and cover plate.

In a third embodiment of the invention the chamber comprises deflectors on both locations of the first and second embodiment of the invention, i.e. at least one deflector is located between at least one opening and the cylinder and at least one opening of a second tube and the rotational axis of the cylinder. The location of the deflectors with respect to the openings and/or the distance to the cylinder can be the same or different. Fig.2 shows this embodiment of the invention with opening (7, 8) and deflector (6, 9). In this embodiment, the chamber may be provided with 2, 4, 6 or 8 openings in both the base and cover plate.

In the third embodiment of the invention, the two openings of the chamber are connected or give access to two different volumes of the chamber and can be used to drain the liquid provided therein. With the shielding effect of the deflectors of the invention, re-mixing of the layers during draining is reduced, resulting in higher purity of the fractions obtained by draining the layers and/or the enablement of higher draining speed.

The deflectors utilized in the present invention are preferable substantially parallel with the cylinder i.e. are bended according to the curve radius of the cylinder. The shape of the deflector can be rectangular, triangular, half-round or elliptic. In a preferred embodiment, the deflector has a broad base located at the opening of a tube and a smaller peak area in order to apply lower shear forces to the cells during centrifugation. The edges of the deflector should be chamfered to avoid cell losses by cutting or ripping of the cellular membrane at the edges. Most preferred, the deflector is half-round or half-elliptic.

The size of the deflectors should be sufficient to reduce the unwanted volume flow, for example by draining liquid from the volume of the chamber located behind the opening in direction to the outside of the chamber when the draining of the part of the chamber located towards the rotational axis is desired.

The size of the deflectors depends on the volume of the chamber, the volume to be shielded and the intended draining speed. For example, the larger the volume to be shielded, the larger the deflector should be. Regardless of its shape, the deflector should have a width at the base (at the opening of a tube) of 1/10 to 1/60, preferable of 1/25 to 1/40 of the inner circumference of the cylinder. For example, a cylinder having an inner diameter of 10 cm is provided with deflectors having a width at the base (at the opening of a tube) of 1 to 2 cm.

The height of the deflector is preferable the same (100%) or smaller, like 50 to 95% of the width at the base.

The size of the surface of a deflector can be calculated or estimated from the given ranges in width and height, but is usually between 0.1 cm² and 10 cm². In any case, the size of the deflector should not hamper the separation of the sample into layers but to reduce unwanted flow of liquid from the separated layers.

The chamber of the invention may comprise several deflectors, which may have the same or different size and/or height and/or width.

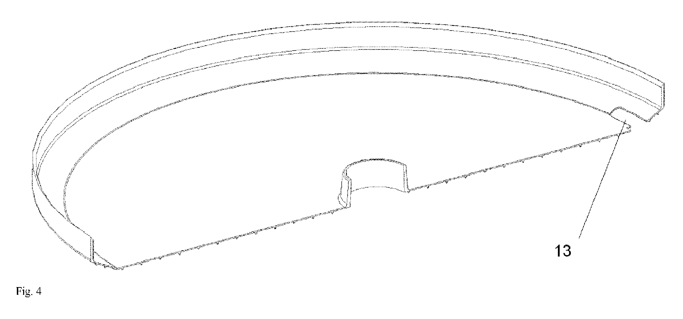

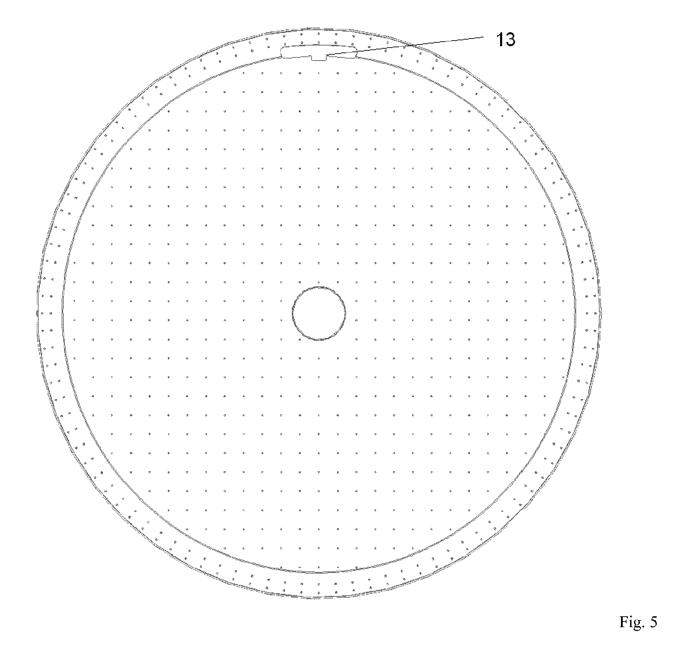

If the chamber of the invention is provided with deflectors at the openings, the gas-permeable membrane and the support structure are provided with orifices or openings for the deflectors and the openings. Gas-permeable membranes with openings for deflectors are marked with (13) in Fig. 4 and 5.

A centrifugation chamber according to the invention preferably comprises as shown in Fig. 2 and 3, a rotational axis with a rotating seal and preferably with two fluid lines or input/output ports for liquids (10). The centrifuge chamber comprises furthermore a cover plate (11), a base plate (12) joined by cylinder (5). The base plate (5) is provided with tubes, which are connected to the respective input/output ports (10) and openings (6, 8) into the chamber. The openings (6, 8) are provided with deflectors (7, 9).

In the embodiment shown in Fig. 2 and 3 , the base plate and the cover plate of the chamber are provided with at least one orifice for input (3) and/or output (4) of gases.

The orifices for input and/or output of gases can be provided with a sterile filter. The chamber may comprise 1, 2 or 4 orifices for input and/or output of gases.

### Gas-permeable membrane with functionalized surfaces

In another embodiment of the invention, the gas-permeable membrane may be provided with a functionalized surface i.e. is treated with chemical or physical means or comprise a coating of chemical or physical immobilized bioactive compounds.

The term "functionalized surface of the gas-permeable membrane" as used in this application includes all types of surfaces which can provide a stimulus to a cell. Typically, functionalized surface comprise a coating of chemical or physical immobilized bioactive compounds, like
- proteins, peptides, nucleic acids;
- spacer molecules enhancing the adhesion of cells or bioactive compounds to the cell modifying surfaces like hydrophilic polymers (functionalized poly lactate, polyvinyl alcohols, polysaccharides; functionalized dextranes);
- organic or inorganic particles as carrier of bioactive compounds, especially magnetic particles coated with functionalized poly lactate, polyvinyl alcohols or functionalized dextranes;
- substances enhancing cell adhesion, e.g. polypeptides, lipids, polysaccharides;
- viruses and retroviruses or particles thereof
- cells which can be used for modification of a target cell, such as antigen presenting cells, "accessory cells" producing certain bioactive factors or cell lines transfected with certain functional molecules.
- stimulus provided by mitogens, cytokines, stimulatory antibodies or receptor ligands
- stimulus provided by hydrophilic properties of the surface

In a variant of the invention, the gas-permeable membrane may be functionalized with any substance which is suitable for cell culture and useful or required to introduce preferable cell culture conditions for a given cell type.

The gas-permeable membrane may functionalized by chemically treatment for example with strong bases, oxygen or a mixture of fluorine and oxygen or by physically treatment with corona or plasma to enhance hydrophilic properties.

The gas-permeable membrane can be functionalized in order to enhance adherence and/or proliferation of cells on the cell modifying surfaces. Suitable substances for functionalization of the gas-permeable membrane are glycoproteins, polypeptides, glycosaminoglycans, disccharides, biotin binding molecules or protein tags. For example, the gas-permeable membrane may be coated with extracellular matrix proteins including all collagen types (I to VIII).

Furthermore, the gas-permeable membrane may be functionalized with an affinity binding system. One of the most widely used affinity binding system is the avidin-biotin or streptavidin-biotin system. For example, the cell modifying surface may be first coated with avidin and/or streptavidin (or derivates thereof) to facilitate binding of a biotinylated molecule like a biotinylated antibody. It is furthermore possible to coat the cell modifying surface first with biotin (or derivates thereof) to facilitate binding of another molecule functionalized with streptavidin and/or avidin. Both variants result in high affinity binding of the second molecule to the cell modifying surfaces. The strong interaction between streptavidin or avidin-biotin is made much weaker by using a combination of modified streptavidin or avidin and modified biotin like desthiobiotin or a derivative thereof like DSB-X Biotin (Hirsch et al. 2002: "Easily reversible desthiobiotin binding to streptavidin, avidin, and other biotin-binding proteins: uses for protein labeling, detection, and isolation". Analytical Biochemistry 308: 343-357; US2008/0255004A1). A protein, such as an antibody may be biotinylated with the modified biotin. When this protein is immobilized by binding the modified biotin to an optionally modified streptavidin or avidin molecule bound to the cell modifying surface, it may be released under mild conditions by adding free biotin.

Further affinity binding systems suitable for the cell modifying surfaces comprise antibodies, for example antibodies against biotin or protein tags for example IIsopeptago, BCCP or Myc-tag.

The gas-permeable membrane may be further be coated with libraries of substances synthesized with methods of combinatorial chemistry in order to identify substances which work best as binding system for a given cell type.

Certain bioactive polymers may be used as spacer molecules enhancing the adhesion of cells or the binding of other substances on the gas-permeable membrane like functionalized poly lactic acid, polyvinyl alcohols, polysaccharides or dextranes or derivates thereof. This binding system is especially useful as basic coating of a gas-permeable membrane produced from a hydrophobic plastic material like poly carbonate, polystyrene or polyethylene. The gas-permeable membrane of the membranes may be coated with highly reactive polymers as e.g. disclosed in US 6,977,138B2.

The gas-permeable membrane can comprise one or more substances which enhance adhesion and/or proliferation of cells. Especially useful are one or more substances selected from the group consisting of collagen types (I to VIII), fibronectin, gelatin, laminin, elastin, hyaluronic acid, keratan sulfate, chondroitin sulfate, heparan sulfate proteoglycans, poly-d-lysine, avidin, streptavidin, biotin, antibodies, antibodies against biotin or protein tags, protein tags like IIsopeptag, BCCP, Myc-tag, Calmodulin-tag, FLAG-tag, HA-tag, His-tag, Maltose binding protein-t a g , Nus-tag, Glutathione-S-transferase-tag, Green fluorescent protein-tag, Thioredoxin-tag, S-tag, Softag 1, Softag 3, Strep-tag, SBP-tag, Ty tag, certia, poly lactate, polyvinyl alcohols, polysaccharides and dextran.

In another variant of the invention, cell modification comprises cellular modification like activation, proliferation, dedifferentiation and/or differentiation of cells. Accordingly, the cell modifying surfaces of the gas-permeable membrane may be functionalized with any substance which is suitable for cellular modification of cells like cell activation, proliferation, dedifferentiation and differentiation of cells. The gas-permeable membrane can further be functionalized with particles being functionalized with at least one substance suitable for cellular modification of cells like cell activation, proliferation, dedifferentiation and differentiation of cells.

Particular, cell modification by the method and the device of the invention comprises the alteration of gene expression, protein expression, post-translational or posttranscriptional modifications of genes, mRNAs or proteins, protein phosphorylation, histone modification, or modification of intracellular signaling cascades (e.g. Ca2+ influx).

Furthermore, cellular modification may comprise cell activation for example by agonistic or antagonistic antibodies, cytokines, growth factors, (de-)activating ligands, pharmacologically active substances, mitogens, DNA or RNA-modifying substances.

### Cell processing system

In a further embodiment, the centrifuge of the present invention can be part of a sample processing system, such as known from WO 2009/072006, WO 2009/072003 or EP 0 869 838 Bl, which is hereby incorporated by reference. The sample processing unit can be coupled to the input/ output port of the centrifugation chamber and may comprise a separation column holder, a pump, a plurality of containers for (intermediate) storage of liquids during the separation process and a plurality of valves configured to at least partially control fluid flow through a fluid circuitry and a separation column positioned in the holder.

### Cell culturing with the centrifugation chamber of the invention

Cell culturing requires supply with cell culturing media and gases. Cell culturing media is either supplied in a constant or batch-wise flow via the i/o ports of the chamber or the chamber is filled once with cell media sufficient for the cell culturing process envisaged. Suitabel cell media are known to the person skilled in the art and include one or more of the following media DMEM, HBSS, DPBS, RPMI, Iscove's medium, X-VIVO™, each optionally supplemented e.g. with fetal calf serum, human serum or serum substitutes or other nutrients or cell stimuli like Cytokines. The media can be standard cell media like the above mentioned media or special media for e.g. primary human cell culture (e.g. for endothelia cells, hepatocytes or keratinocytes) or stem cells (e.g. dendritic cell maturation, hematopoietic expansion, keratonocytes, mesenchymal stem cells or T cell expansion). The media may have supplements or reagents well known in the art, e.g. albumins and transport proteins, amino acids and vitamins, antibiotics, attachments factors, growth factors and cytokines, hormones or solubilising agents. Various media are commercially available e.g. from LifeTechnologies or Sigma-Aldrich.

Temperature and gas composition of the centrifugation chamber can be controlled and adjusted if appropriate for the cell types or the modification steps to be performed. For this purpose, a heating and/or cooling means can be attached to the device of the invention. In the method of the invention, the cells to be cultured are supplied with gases such as air, O₂, N₂ and CO₂ by diffusion through the gas-permeable membrane.

### Use of the chamber

The chamber according to the invention can for example be used in the following processes:
- Cell processing like cell activation, cell proliferation, cell transfection, cell staining followed or subsequently to a separation or washing step
- isolating leucocytes by separating and discharging erythrocytes and plasma from human blood
- isolating certain subpopulations of leucocytes, for example leucocytes having one or more of the following surface marker CD4, CD8, CD25, CD 34 and/or CD 133 by separating leucocytes from human blood with subsequent cell labelling
- preparation of leucocytes by discharging erythrocytes and plasma from human blood followed by one or more washing steps with cell media and/or density gradient additives
- isolating, enriching or depleting cells having one or more of the following surface marker CD4, CD8, CD25, CD 34 and/or CD 133 from human blood for use in regenerative medicine, peripheral artery, liver disease or cardiac stem cell therapy

## Claims

1. A centrifuge chamber comprising a cylinder having a base plate and a cover plate, a rotational axis with at least one port for input and/or output of liquids, at least one port for input of gases and at least one layer for cell culturing, **characterized in that** the layer for cell culturing comprises a gas-permeable membrane on which cells can be cultured and wherein the at least one port for input and/or output of gases is connected to the gas-permeable membrane.

2. A centrifuge chamber according to claim 1, **characterized in that** the gas-permeable is applied on a support structure.

3. A centrifuge chamber according to claim 1, **characterized in that** the gas-permeable is applied on the base plate.

4. A centrifuge chamber according to claim 1, **characterized in that** the gas-permeable membrane and/or the support structure and/or the base plate is provided with a plurality of spacer elements.

5. A centrifuge chamber according to claim 2, **characterized in that** the support or the base plate is structure is provided with channels.

6. A centrifuge chamber according to any of the claims 1 to 3, **characterized in that** the gas-permeable membrane is connected to a support structure or the base plate in a gas tight manner.

7. A centrifuge chamber according to any of the claims 1 to 6, **characterized in that** the gas-permeable membrane and the support structure having a optical transparence suitable for optical microscopy.

8. A centrifuge chamber according to any of the claims 1 to 7, **characterized in that** the surface of the gas-permeable membrane is functionalized for cell culturing.

9. A centrifuge chamber according to any of the claims 1 to 8, **characterized in that** the at least one port for input and/or output of liquids is connected to openings located in the base plate and/or cover plate into the centrifuge chamber and at least one opening is provide with at least one deflector having a width at its base of at most 1/10 of the inner circumference of the cylinder.

10. A centrifuge chamber according to claim 9, **characterized in that** at least one deflector is located between at least one opening and the cylinder.

11. A centrifuge chamber according to claim 9, **characterized in that** at least one deflector is located between at least one opening and the rotational axis of the cylinder.

12. A centrifuge chamber according to claim 9, **characterized in that** at least one first deflector is located between at least one opening and the rotational axis of the cylinder and at least one second deflector is located between at least one opening and the cylinder.

13. A centrifuge chamber according to any of the claims 9 to 12, **characterized in that** the deflector is substantially parallel with the cylinder and has a width of 5 to 50 times the width of the opening it is located at.
